Europäisches Patentamt

European Patent Office

Office européen des brevets

(19)

(11) Numéro de publication: **0 296 950 B1**

# FASCICULE DE BREVET EUROPEEN

(12)

(45) Date de publication de fascicule du brevet: **26.08.92**

(51) Int. Cl.⁵: **B65D 83/14**, A61M 11/06

(21) Numéro de dépôt: **88401526.4**

(22) Date de dépôt: **20.06.88**

(54) **Pulvérisateur de produits stériles, notamment de solutions aseptiques pour traitements médicaux et chirurgicaux.**

(30) Priorité: **22.06.87 FR 8708705**

(43) Date de publication de la demande:
**28.12.88 Bulletin 88/52**

(45) Mention de la délivrance du brevet:
**26.08.92 Bulletin 92/35**

(84) Etats contractants désignés:
**AT BE CH DE ES FR GB GR IT LI LU NL SE**

(56) Documents cités:
**WO-A-86/01731**
**BE-A- 897 240**
**DE-A- 2 010 650**
**FR-A- 1 074 398**
**FR-A- 2 437 217**

(73) Titulaire: **CENTRE REGIONAL DE TRANSFU-SION SANGUINE DE LILLE**
**19-21 rue Camille Guérin**
**F-59012 Lille(FR)**

(72) Inventeur: **Thiebaut, Jean-Marc**
**9 rue Paul Cézanne**
**F-59120 Loos(FR)**

(74) Mandataire: **Lhuillier, René et al**
**ARMENGAUD JEUNE CABINET LEPEUDRY 52,**
**avenue Daumesnil**
**F-75012 Paris(FR)**

## Description

L'invention concerne un pulvérisateur de produits stériles comme décrit dans la première partie de la revendication 1, et plus particulièrement de solutions aseptiques pour traitements médicaux et chirurgicaux comme par exemple des colles stériles.

Pour le traitement de la peau, notamment la reconstitution de la peau des personnes brûlées ou d'une façon plus générale pour des interventions chirurgicales à tissu épidermique ou parenchymateux, il existe des solutions obtenues par exemple à partir de thrombine calcique mélangées à un fibrinogène, qui doivent être appliquées sur les zones malades dans des conditions parfaites d'aseptie . Pour cela on utilise des dispositifs de pulvérisation qui permettent une meilleure répartition du produit et sont moins traumatisants pour le malade. Un problème se pose toutefois du fait que ces produits résultant du mélange de deux constituants doivent être préparés de façon extemporanée, quelques instants avant le traitement. Pour cela on pourrait simplement verser dans un premier flacon contenant la solution de thrombine calcique, le contenu d'un deuxième flacon contenant le fibrinogène, puis mélanger ces deux constituants et les verser dans un flacon pulvérisateur, toutes ces opérations devant bien entendu être assurées en atmosphère stérile, et avec les plus grandes précautions. Comme ce genre de manipulation est malaisé, on pourrait utiliser un ensemble mélangeur qui évite le débouchage de flacons et le transfert de liquides de l'un à l'autre, et qui mettrait en oeuvre par exemple un flacon à deux compartiments superposés, adaptables l'un à l'autre, et des moyens permettant d'instiller le contenu de l'un, dans la solution de l'autre, par exemple à l'aide d'un organe déformable. Cet ensemble n'exclut pas cependant de devoir assurer le transfert à un récipient de pulvérisation, ou l'adaptation d'un pulvérisateur à cet ensemble ; il y a donc encore un risque de renversement du produit, ou tout du moins de ne pas assurer la manipulation dans des conditions parfaitement stériles. De plus comme les deux composants mélangés induisent une réaction de polymérisation, une prise en masse du mélange est possible.

On connaît par ailleurs, notamment par le BE-A-897 240 une technique d'injection d'un ingrédient dans un récipient dont le fond est percé d'un trou obturé par un joint élastique pouvant être percé par une aiguille pour l'injection de fluide. Mais l'organe perforable en question n'a pas de configuration particulière et le récipient ne permet pas de récupérer aisément les dernières gouttes de liquide.

Pour s'affranchir de ces inconvénients, il existe un système de pulvérisation constitué de deux seringues, chacune remplie d'un des fluides à mélanger, montées sur un même support, dont les embouts d'extrêmité coopèrent avec une tête de pulvérisation destinée à projeter simultanément les deux produits. Mais l'ensemble que constituent les seringues, leur support et la tête spéciale de pulvérisation, est un produit spécifique coûteux et d'utilisation difficile. En outre ce système nécessite une infrastructure environnante, ce qui le rend inutilisable en dehors de salles médicales appropriées ou de blocs opératoires, puisque des arrivées de gaz, des détendeurs et tous dispositifs de commande adaptés sont nécessaires.

Un des buts de l'invention a donc été de créer un pulvérisateur de produits stériles, qui soit simple à utiliser, qui soit également maniable et peu coûteux à réaliser, dont le remplissage peut être assuré de façon stérile et qui permet d'assurer la pulvérisation d'un produit préparé de façon extemporanée, pour tout traitement médical et chirurgical, et qui évite ainsi toute infrastructure de mise en oeuvre. Dans le cas des applications de colle biologique, ce pulvérisateur permet la pulvérisation séquentielle et indépendante des solutions de fibrinogène d'une part et de thrombine calcique d'autre part induisant sur le tissu traité l'apparition rapide d'un flux de fibrine résistant.

Un objet de la présente invention consiste donc en un pulvérisateur de produits stériles comportant un flacon destiné à recueillir la solution à pulvériser et sur un orifice duquel est ou peut être rapportée une tête de distribution permettant la pulvérisation du liquide par un agent propulseur ou par l'action mécanique du piston d'une pompe à précompression, ledit flacon étant muni d'un autre orifice localisé à sa partie inférieure et obturé de façon étanche par un organe en matière souple perforable et restant étanche après pulvérisation, pulvérisateur selon lequel l'organe perforable est un bouchon présentant une partie inférieure en forme de disque correspondant au diamètre de l'orifice et une partie supérieure se développant à l'intérieur du flacon, et qui est percée d'un puits central borgne pour la récupération des dernières gouttes contenues dans le récipient en vue de sa pulvérisation.

De plus le bouchon est muni d'échancrures latérales verticales s'étendant sur toute la hauteur de la partie supérieure et sur les côtés du bouchon, échancrures qui communiquent par des canaux transversaux, avec le puits central.

Avantageusement, le flacon se présente comme un tube à deux cols et deux orifices opposés de mêmes dimensions obturés tous les deux par un bouchon obturateur identique, l'un des deux pouvant être percé et équipé d'un dispositif de pulvérisation.

D'autres avantages de l'invention apparaîtront à la lecture de la description qui va suivre d'exemples non limitatifs de réalisation dans lesquels il est fait référence aux dessins annexés qui représentent :

- figure 1 : une vue schématique en coupe d'un pulvérisateur ;
- figure 2 : une vue en coupe à plus grande échelle selon II-II de la figure 3 ;
- figure 3 : une vue du dessus du bouchon inférieur ; et
- figure 4 : une variante de flacon de pulvérisation.

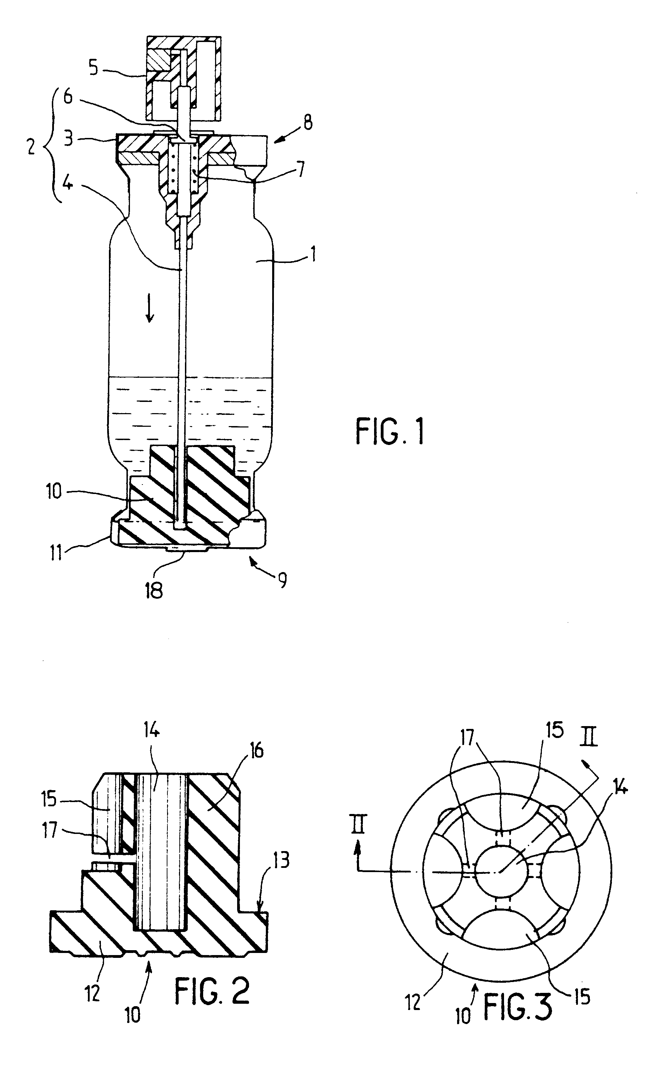

On a représenté à la figure 1 un pulvérisateur du type aérosol qui comporte esentiellement un flacon 1 dont l'orifice supérieur 8 est fermé de façon étanche par une pompe à précompression 2. La pompe comporte essentiellement une coupelle 3 sertie sur le rebord de l'orifice 8, un tube plongeur 4 qui s'étend jusqu'à la partie inférieure du flacon et qui communique vers le haut avec une tête de distribution 3 et un petit clapet obturateur 6. La tête, qui dispose de façon habituelle de buses de pulvérisation appropriées, commande l'ouverture du petit clapet 6 à l'encontre de la force d'un ressort 7, ce qui permet le passage de liquide du tube 4 vers la tête sous l'action d'une pression exercée sur la tête de la pompe.

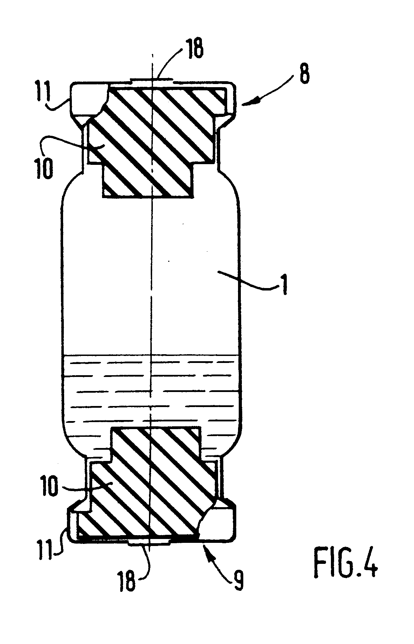

Le flacon 1 est muni à sa partie inférieure d'un autre orifice 9 fermé par un bouchon 10. Ce bouchon de structure particulière, est représenté en détail aux figures 2 et 3. Il obture de façon étanche l'orifice 9 et il est maintenu en place par une coupelle annulaire de sertissage 11 prenant sur le rebord de cet orifice, coupelle qui ne recouvre pas toute la surface du bouchon et laisse apparente la partie centrale de ce dernier. Une capsule 18 assurant l'inviolabilité est sertie sur cette coupelle annulaire. Le bouchon est réalisé en matière souple et perforable présentant la propriété d'assurer une bonne étanchéité le long d'un organe perforateur, et de reprendre sa forme initiale après perforation, en restant étanche. La partie inférieure 12 du bouchon 10, en forme de disque, correspondant au diamètre de l'orifice 9, est maintenue par un épaulement 13 entre la coupelle 11 et le rebord de l'orifice 9. La partie supérieure 16 du bouchon qui se développe à l'intérieur du flacon est percée d'un puits central 14 dont le fond est sensiblement au niveau du disque 12. Quatre échancrures 15 de profil arrondi s'étendent sur toute la hauteur et sur les côtés du bouchon, à 90° les unes des autres, parallèlement au puits 14. Chacune est traversée, sensiblement à mi-hauteur par des canaux 17 qui font communiquer les échancrures avec le puits 14. On notera que la partie inférieure du flacon 1 dans la zone périphérique au bouchon 10, c'est-à-dire la partie basse du flacon, présente un profil en forme d'entonnoir permettant la récupération des dernières gouttes du liquide vers le fond du flacon au niveau du puits central 14. L'extrémité inférieure du tube 4 pénètre dans ce puits central et débouche dans cette zone centrale inférieure du bouchon.

Avant de pouvoir vaporiser un mélange, avec ce pulvérisateur, on le prépare de façon extemporanée et on l'introduit dans le flacon dont on a ôté préalablement la capsule en piquant au travers du bouchon 10. Après retrait de la seringue, le bouchon 10 s'est refermé et reste étanche ; on peut pulvériser le mélange par action sur la tête de distribution 5. Compte tenu de la structure du bouchon 10, les dernières gouttes du produit, rassemblées au fond du puits 14, pourront être récupérées par le tube 4 et vaporisées.

Dans le cas où on peut disposer sur place d'une source d'azote, quand le traitement doit être effectué par exemple dans un hôpital, on peut évidemment effectuer une alimentation du flacon avec un gaz propulseur. Pour cela, le bouchon inférieur 10 du pulvérisateur serait traversé de façon étanche par un conduit d'alimentation en gaz propulseur, par exemple de l'azote, provenant d'un générateur.

Dans une variante de réalisation illustrée à la figure 4, le flacon se présente comme un tube à deux cols et deux orifices opposés 8 et 9, avantageusement de même dimension pour que l'orifice supérieur 8 du flacon 1 puisse être fermé par un bouchon 10 identique au bouchon fermant l'orifice inférieur 9 et décrit précédemment en référence aux figures 2 et 3, lequel bouchon supérieur étant également profilé pour permettre la récupération du liquide, et étant équipé de la même coupelle de sertissage 11, et de la capsule de fermeture 18.

Ledit flacon peut être vide ou rempli d'un liquide ou produit lyophilisé et stérile.

Quand on veut pulvériser un liquide à partir d'un tel flacon qui n'est pas prérempli de liquide, on ôte la capsule 18 d'un des deux bouchons qui est le bouchon supérieur puis on le perce à l'aide d'une aiguille, qui comme le tube 4 du premier exemple, fait partie d'un dispositif de pulvérisation non représenté qui vient s'encliqueter sur le col du flacon en coiffant le bouchon supérieur et l'orifice 8. L'extrémité de ladite aiguille vient alors dans le puits central du bouchon inférieur, comme le tube plongeur 4.

Puis après avoir ôté la capsule 18 du bouchon inférieur 10, on introduit à l'aide d'une seringue le liquide que l'on désire pulvériser en piquant au travers du bouchon. Après retrait de la seringue le bouchon s'est refermé et reste étanche. L'appareil est alors prêt pour une pulvérisation puisque le dispositif a été fixé sur le bouchon supérieur.

L'ensemble du système de pulvérisation ayant été stérilisé, les risques de manipulation dans des conditions non parfaitement stériles sont éliminés.

## Revendications

1. Pulvérisateur de produits stériles, notamment de solutions aseptiques pour traitements médicaux et chirurgicaux comportant un flacon (1) destiné à recueillir la solution à pulvériser et sur un orifice (8) duquel est ou peut être rapportée une tête de distribution (5) de la solution pulvérisée par un agent propulseur ou par l'action mécanique du piston d'une pompe à précompression, ledit flacon étant muni d'un autre orifice (9) localisé à sa partie inférieure, obturé de façon étanche par un organe en matière souple, perforable et restant étanche après perforation, caractérisé en ce que l'organe perforable est un bouchon (10) présentant une partie inférieure (12) en forme de disque correspondant au diamètre de l'orifice (9) et une partie supérieure (16) qui se développe à l'intérieur du flacon et est percée d'un puits central borgne (14) pour la récupération des dernières gouttes contenues dans le récipient en vue de sa pulvérisation.

2. Pulvérisateur selon la revendication 1, caractérisé en ce que le bouchon (10) est muni d'échancrures latérales verticales (15) de profil arrondi s'étendant sur toute la hauteur de la partie supérieure et sur les côtés du bouchon.

3. Pulvérisateur selon la revendication 2, caractérisé en ce que chaque échancrure (15) est traversée, sensiblement à mi-hauteur par des canaux (17) qui les mettent en communication avec le puits central (14).

4. Pulvérisateur selon les revendications 1 et 2, caractérisé en ce que la partie inférieure du flacon (1) dans la zone périphérique au bouchon (10) présente un profil en forme d'entonnoir ramenant les dernières gouttes de liquide au niveau du puits central (14).

5. Pulvérisateur de produits stériles selon la revendication 1, caractérisé en ce que le flacon (1) se présente comme un tube à deux cols et deux orifices opposés (8,9) de mêmes dimensions obturés tous les deux par un bouchon obturateur identique (10), l'un des deux pouvant être percé et équipé d'un dispositif de pulvérisation.

## Claims

1. A spray for sterile products, especially aseptic solutions used in medical and surgical treatments, comprising a flask (1) for collecting the solution to be sprayed, on one orifice of which flask there is or may be fixed a distribution head (5) for the solution sprayed by means of a propellant or by the mechanical action of the piston of a precompression pump, said flask having another orifice (9) located in its lower part and closed so as to be sealed by a member made from a perforable flexible material which remains sealing after perforation, characterised in that the perforable member is a stopper (10) having a lower part (12) in the form of a disk corresponding to the diameter of the orifice (9), and an upper part (16) which extends inside the flask and through which passes a blind central well (14) for the recovery of the last drops contained in the container for the purpose of spraying those drops.

2. A spray according to claim 1, characterised in that the stopper (10) is provided with vertical lateral indentations (15) with rounded profile extending over the whole height of the upper part and along the sides of the stopper.

3. A spray according to claim 2, characterised in that each indentation (15) has passing therethrough, substantially at mid-height, channels (17) which place them in communication with the central well (14).

4. A spray according to claims 1 and 2, characterised in that the lower part of the flask (1) in the zone peripheral to the stopper (10) has a profile in the form of a funnel collecting the last drops of liquid at the level of the central well (14).

5. A spray for sterile products according to claim 1, characterised in that the flask (1) is in the form of a tube with two necks and two opposite orifices (8, 9) of the same dimensions, each of which is closed by an identical closing stopper (10) and one of which may be pierced and fitted with a spray device.

## Patentansprüche

1. Zerstäuber für sterile Produkte, insbesondere für aseptische Lösungen zur medizinischen und chirurgischen Behandlung, umfassend einen Behälter (1), welcher zur Aufnahme der zu pulverisierenden Lösung bestimmt ist, und auf einer Öffnung (8) desselben ist ein Verteilerkopf (5) für die durch ein Treibmittel oder durch die mechanische Wirkung des Kolbens einer Vorverdichtungspumpe zerstäubte Lösung aufgesetzt oder kann aufgesetzt sein, wobei der Behälter mit einer weiteren Öffnung (9) versehen ist, welche sich an seinem Unterteil befindet, und durch ein nachgiebiges, durchbohrbares und nach Durchbohrung dicht bleibendes Mittel dicht verschlossen ist, dadurch gekennzeichnet, daß das durchbohrbare Mittel ein Stopfen (10) ist, welcher ein Unterteil (12) in Form einer Scheibe mit einem der Öffnung (9) entsprechenden Durchmesser und ein Oberteil (16) aufweist, welches sich in das Innere des Behälters erstreckt und zur Gewinnung der letzten, in dem Behälter enthaltenen Tropfen zu ihrer Zerstäubung mit einer zentralen Blindbohrung (14) durchbohrt ist.

2. Zerstäuber gemäß Anspruch 1, dadurch gekennzeichnet, daß der Stopfen (10) mit seitlichen, senkrechten Aussparungen (15) mit abgerundetem Profil versehen ist, welche sich über die gesamte Stärke des Oberteils und über die Seiten des Stopfens erstrecken.

3. Zerstäuber gemäß Anspruch 2, dadurch gekennzeichnet, daß
jede Aussparung (15) genau auf halber Höhe von Kanälen geschnitten wird, welche sie mit der zentralen Bohrung (14) verbinden.

4. Zerstäuber gemäß den Ansprüchen 1 und 2, dadurch gekennzeichnet, daß das Unterteil des Behälters (1) in der Umfangszone am Stopfen (10) ein Profil in Form eines Trichters aufweist, welches die letzten Tropfen der Flüssigkeit auf das Niveau der zentralen Bohrung zurückführt.

5. Zerstäuber für sterile Produkte gemäß Anspruch 1, dadurch gekennzeichnet, daß der Behälter (1) ein Rohr mit zwei gegenüberliegenden Hälsen und Öffnungen (8, 9) mit gleichen Abmessungen darstellt, die alle beide durch einen identischen Verschlußstopfen (10) verschlossen sind, wobei einer von beiden durchbohrt und mit einer Zerstäubungsvorrichtung ausgestattet sein kann.

FIG. 1

FIG. 2

FIG. 3

FIG.4